(19) Europäisches Patentamt / European Patent Office / Office européen des brevets

(11) **EP 3 960 025 B1**

(12) **EUROPEAN PATENT SPECIFICATION**

(45) Date of publication and mention
of the grant of the patent:
**21.02.2024   Bulletin 2024/08**

(21) Application number: **20794108.9**

(22) Date of filing: **14.04.2020**

(51) International Patent Classification (IPC):
**A45D 44/22** (2006.01)     **B32B 5/26** (2006.01)
**D04H 1/4374** (2012.01)     **D04H 3/16** (2006.01)
**A41D 13/11** (2006.01)

(52) Cooperative Patent Classification (CPC):
**D04H 3/16; B32B 5/022; B32B 5/08; B32B 5/268;
B32B 5/269; D04H 1/4258; D04H 1/435;
D04H 1/4358; D04H 1/4374; D04H 1/492;
D04H 1/56;** A45D 44/002; B32B 2250/20;
B32B 2260/023; B32B 2262/0215;     (Cont.)

(86) International application number:
**PCT/JP2020/016464**

(87) International publication number:
**WO 2020/218092 (29.10.2020 Gazette 2020/44)**

(54) **FIBER LAYERED BODY AND PRODUCTION METHOD THEREFOR**

FASERSCHICHTKÖRPER UND HERSTELLUNGSVERFAHREN DAFÜR

CORPS STRATIFIÉ DE FIBRES ET SON PROCÉDÉ DE PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.04.2019   JP 2019084945**

(43) Date of publication of application:
**02.03.2022   Bulletin 2022/09**

(73) Proprietor: **Kuraray Kuraflex Co., Ltd.
Okayama-shi, Okayama 702-8045 (JP)**

(72) Inventors:
• **TOMOI, Masanori
Okayama-shi, Okayama 702-8045 (JP)**
• **MATSUO, Akihiro
Okayama-shi, Okayama 702-8045 (JP)**
• **OCHIAI, Toru
Okayama-shi, Okayama 702-8045 (JP)**
• **SHIRAISHI, Ikuhisa
Saijo-shi, Ehime 793-8585 (JP)**

(74) Representative: **Müller-Boré & Partner
Patentanwälte PartG mbB
Friedenheimer Brücke 21
80639 München (DE)**

(56) References cited:
EP-A1- 2 483 078          WO-A1-2010/064710
WO-A1-2010/064710      WO-A1-2018/143430
CN-U- 207 493 504         JP-A- S 602 273
JP-A- H0 226 973          JP-A- H0 760 886
JP-A- 2002 356 958        JP-A- 2003 082 568
JP-A- 2009 121 014        JP-A- 2009 256 856
JP-A- 2013 128 743        KR-B1- 101 902 546
US-A1- 2014 332 476       US-B2- 8 748 693

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
B32B 2262/023; B32B 2262/0292; B32B 2262/04;
B32B 2262/144; B32B 2262/16; B32B 2305/70;
B32B 2307/5825; B32B 2307/718; B32B 2307/726;
B32B 2307/728; B32B 2307/73; B32B 2307/744;
B32B 2307/748; B32B 2432/00; B32B 2535/00;
B32B 2555/00; B32B 2555/02; B32B 2571/00

**Description**

FIELD OF THE INVENTION

The present invention relates to a fiber layered body comprising an extra-fine fiber layer and a non-extra-fine fiber layer, and a method for producing the same.

BACKGROUND OF THE INVENTION

Conventionally, skin care sheets infused or impregnated with cosmetics or other liquids (liquid-containing sheets for applying to living body) have been used as sheets to be placed on epidermis (skin) of human body. In recent years, a wide variety of skin care sheets, such as face masks, have been developed because they can easily maintain the skin in a highly hydrated state. Generally, woven and nonwoven fabrics made of fibers are used as materials for sheets, and nonwoven fabrics are widely used because of their cost. Spunlaced nonwoven fabrics made mainly of cellulosic fibers such as cotton, which has high hydrophilicity, are often used as nonwoven cosmetics-infusion sheets. However, the spunlaced nonwoven fabrics made of cellulosic fibers are insufficient from the viewpoint of irritation to skin as well as matching to contours.

In order to improve above defects, a nonwoven layered body comprising a liquid retention layer and an adhesion layer has been known as a skin-contact sheet excellent in adhesion property and liquid retention (Patent Document 1). In addition, a sheet with a thermoplastic elastomeric fiber layer has been proposed to obtain a lift effect to reduce loosening of skin (Patent Documents 2 and 3). Patent Document 2 discloses a coating sheet in which a layered body of a thermoplastic elastomeric fiber layer and a short fiber layer is integrated with a cellulosic fiber layer by hydroentanglement or needle-punching. In addition, Patent Document 3 discloses an elastic layered sheet in which an extra-fine fiber layer containing elastomeric long fibers is disposed to a hydrophilic short fiber layer by partial thermocompression.

EP 2 483 078 A1 discloses method for forming a multilayer meltblown composite.

CONVENTIONAL ART DOCUMENT

PATENT DOCUMENT

[Patent Document 1] WO 2011/004834
[Patent Document 2] Japanese Patent Laid-open Publication No. 2010-155454
[Patent Document 3] Japanese Patent Laid-open Publication No. 2009-256856

SUMMARY OF THE INVENTION

PROBLEMS TO BE SOLVED BY THE INVENTION

However, in Patent Document 1, in the case where the adhesion layer is made of thermoplastic elastomeric fibers, the nonwoven layered body deteriorates in peeling strength between the adhesion layer and the liquid retention layer due to the shrinkage of the thermoplastic elastomeric fibers. Accordingly, when the nonwoven fabric layered body is elongated before use, there has been a possibility such that the adhesion layer peels off from the liquid retention layer. In the method of Patent Document 2, although the elasticity caused by the thermoplastic elastomeric fiber layer can be attained, since Patent Document 2 requires hydroentanglement or needle-punching to integrate the layered body of the thermoplastic elastomeric fiber layer and the short fiber layer with the cellulosic fiber layer, the thermoplastic elastomeric fiber layer itself easily peels from the short fiber layer. Further, in the method disclosed in Patent Document 3, since the fiber layers are integrated by the partial thermocompression with forming dents at the pressure bonding points, when the elastic layered sheet is used as a liquid-infused sheet, the sheet is prone to insufficient adhesion to the skin at start of use.

Therefore, an object to be solved by the present invention is to provide a material not only having a high peeling strength between layers but also being capable of maintaining a liquid at an appropriate amount to be retained, while adhering to the skin and exerting lift (tightening) effect.

MEANS TO SOLVE PROBLEMS

As a result of intensive studies to solve the aforementioned problems, the inventors have found, as a novel problem, that (i) where an extra-fine fiber layer comprising thermoplastic elastomeric fibers is layered with a non-extra-fine fiber layer, the elastomeric feature of the thermoplastic elastomer fibers makes it difficult to integrate the extra-fine fiber layer with the non-extra-fine fiber layer. In order to improve this problem, the inventors has further found that (ii) peeling strength between the extra-fine fiber layer comprising thermoplastic elastomeric fibers and the non-extra-fine fiber layer can be improved both by making a part of the fibers constituting the extra-fine fiber layer to enter into the non-extra-fine fiber layer at the time of bonding the extra-fine fiber layer with the non-extra-fine fiber layer, and by making the fibers constituting the extra-fine fiber layer to be substantially melted in contact to the interface with the non-extra-fine fiber layer. Moreover, the inventors further has found that (iii) a fiber layered body with such peeling strength between layers can achieve an integrated structure as a fiber layered body without embossing that causes deep surface unevenness for integrated structure; and further can have an improved adhesion to skin due to flat surface unevenness. In the light of the above findings, the inventors have achieved the present invention.

EFFECT OF THE INVENTION

According to the present invention, even though thermoplastic elastomeric fibers constitute an extra-fine fiber layer of a fiber layered body, the fiber layered body has excellent peeling strength between the extra-fine fiber layer and a non-extra-fine fiber layer adjacent to the extra-fine fiber layer. Further, integration of thermoplastic elastomeric fibers with the non-extra-fine fiber layer makes it possible to obtain a fiber layered body excellent in entire stretchability as well as adhesion, which result in providing a fiber layered body capable of exerting lifting or tightening effect.

DESCRIPTION OF THE EMBODIMENTS

Fiber Layered Body

A fiber layered body (fiber laminate) according to the present invention comprises: an extra-fine fiber layer which comprises thermoplastic elastomeric fibers and has an average fiber diameter of less than 10 $\mu$m; and a non-extra-fine fiber layer which comprises fibers having an average fiber diameter of 10 to 30 $\mu$m and is adjacent to the extra-fine fiber layer. The fiber layered body has a peeling strength between the extra-fine fiber layer and the non-extra-fine fiber layer of 0.50 N/5cm or higher, and a surface unevenness based on a thickness of the fiber layered body of 40% or less.

The extra-fine fiber layer and the non-extra-fine fiber layer are adjacent to each other, and some of the thermoplastic elastomeric fibers that constitute the extra-fine fiber layer preferably enter into inner side of the non-extra-fine-fiber and act as anchors. Further, a part of the thermoplastic elastomeric fibers may have portions each being deformed in conformity to the non-extra-fine-fiber shapes at the interface with the non-extra-fine fiber layer, and may preferably be integrated with the non-extra-fine fiber layer at the fiber level. Since the fiber layered body according to the present invention has excellent integration between the extra-fine fiber layer and the non-extra-fine fiber layer, it is possible to achieve a desirable peeling strength between the extra-fine fiber layer and the non-extra-fine fiber layer even without interposing a binder layer therebetween.

In the fiber layered body according to the present invention, it is sufficient if the fiber layered body comprises at least the above-mentioned double layer structure of the extra-fine fiber layer and the non-extra-fine fiber layer, for example, the fiber layered body may have a three-layer structure in which both sides of the non-extra-fine fiber layer are disposed with the thermoplastic elastomeric extra-fine fiber layers, respectively. Further, the fiber layered body according to the present invention may comprise additional layer(s). Examples of the additional layers include a film layer, a binder layer, and a fiber layer. These layers, alone or in combination, may be arranged on at least one side of the fiber layered body.

The fiber layered body according to the present invention comprises an extra-fine fiber layer formed from a thermoplastic elastomer, and a non-extra-fine fiber layer integrated with the extra-fine fiber layer, so that the fiber layered body has an excellent integrated structure as a fiber layered body. In such a fiber layered body, the extra-fine fiber layer and the non-extra-fine fiber layer do not peel off from each other even when the fiber layered body is stretched before application. Moreover, since the fiber layered body according to the present invention comprises a thermoplastic elastomeric extra-fine fiber layer and has reduced unevenness, where the fiber layered body infused with liquid components is applied to skin with stretching, the fiber layered body (preferably, the extra-fine-fiber layer) adheres to the skin; the cosmetic liquid retained in the non-extra-fine fiber layer is effectively released; and further stretchability of the fiber layered body sheet attains lifting or tightening effect.

Non-Extra-Fine Fiber Layer

The non-extra-fine fiber layer used in the present invention comprises fibers having an average fiber diameter of 10 to 30 $\mu$m. The average fiber diameter of the fibers constituting the non-extra-fine fiber layer is preferably 10 to 25 $\mu$m, and more preferably 10 to 20 $\mu$m. The fibers constituting the non-extra-fine fiber layer generally have a larger fiber diameter than the fibers constituting the extra-fine fiber layer. Where the average fiber diameter of the fibers constituting the non-extra-fine fiber layer is less than 10 $\mu$m, the liquid retention property of the fiber layered body decreases due to reduced bulkiness, whereas the average fiber diameter of the fibers constituting the non-extra-fine fiber layer exceeds 30 $\mu$m, the web becomes stiff such that the sheet becomes uncomfortable to use. It should be noted that, in this specification, the average fiber diameter means the number average fiber diameter of single fibers.

The form of the non-extra-fine fiber layer is not particularly limited to a specific one, and examples thereof may include woven fabrics, knitted fabrics, nonwoven fabrics, and webs. Among them, dry-laid nonwoven fabrics, extrusion (direct-spun) nonwoven fabrics (for example, spunbonded nonwoven fabrics) and the like are preferably used from the viewpoints of productivity, sheet stretchability, and handleability. The non-extra-fine fiber layer may be used singly or in combination of two or more types.

The fibers constituting the dry-laid nonwoven fabric may have a fiber length of about 15 to 70 mm, preferably about 20 to 65 mm, more preferably about 30 to 60 mm, and even more preferably about 35 to 55 mm. With such a fiber length, it is possible to distinguish dry-laid nonwoven fabrics from wet-laid nonwoven fabrics (the wet-laid nonwoven fabrics usually have fiber length of 10 mm or shorter).

For example, in order to produce a dry-laid nonwoven fabric, a web is formed from a predetermined fiber aggregate by a card method or an air-laid method. Thus-obtained web is subjected to binding process for bonding fibers so as to make a resultant to have a practical strength. As the bonding process, there may be mentioned chemical bonding (for example, chemical bonding process), thermal bonding (for example, thermal bonding process, steam-jet process), and mechanical bonding (for example, spunlacing process, needle-punching process), and others. In view of convenience, it is preferred to employ the spunlacing process in which entanglement of fibers is carried out by hydroentanglement process.

In the case of the spunlacing process, short fibers (cut fibers or staple fibers), for example, a mixture of hydrophobic short fibers and hydrophilic short fibers may be opened by carding using a carding machine, for example, to prepare a nonwoven fabric web. Such a nonwoven fabric web may be a parallel web in which orientation degrees of fibers constituting the web are such that fibers are aligned in the machine direction of the carding machine, a cross web in which the parallel web is cross-laid, a random web in which fibers constituting the web are arranged randomly, or a semi-random web in which orientation degrees of fibers constituting the web are in the medium degree between the parallel web and the random web. Considering that entanglement of fibers in the lateral direction of random webs and cross webs may hinder elongation of webs in the lateral direction such that less elongation avoids to match skin contour during use, parallel webs and semi-random webs are preferred because parallel webs and semi-random webs can ensure softness and stretchability of the layered sheet in the lateral direction.

More specifically, examples of nonwoven fabrics may include chemical-bonded nonwoven fabrics, thermal-bonded nonwoven fabrics, spunlaced (hydro-entangled) nonwoven fabrics, needle-punched nonwoven fabrics, air-laid nonwoven fabrics, steam-jet nonwoven fabrics, and spunbonded nonwoven fabrics, and the like. Of these, preferable one may include spunlaced nonwoven fabrics and steamjet nonwoven fabrics from the viewpoint of water retention ratio, sheet stretchability, and others.

The non-extra-fine fiber layer has a wettability necessary to be infused with liquid components, including beauty or medical (efficacy) ingredients (for example, hydrating or moisturizing, cleansing, antiperspirant, fragrant, whitening, blood circulation promoting, cooling, ultraviolet light preventing, skin itch inhibiting, etc.). The non-extra-fine fiber layer also has void spaces necessary to retain such liquid components. Accordingly, it is preferable that the non-extra-fine fiber layer has a role to keep liquid components therein without leaking upon handling the fiber layered body for usage before placing in on a desired body part (for example, face), and to gradually transfer the liquid components toward the extra-fine fiber layer located on the skin side once the fiber layered body is placed on the skin or left thereon.

The fibers constituting the non-extra-fine fibers can be selected depending on the intended use, and from the viewpoint of liquid retention, it is preferable to contain hydrophilic fibers. The inclusion of hydrophilic fibers makes it possible to easily absorb liquid into the layered body when the fiber layered body is infused with liquid components such as cosmetics. Moreover, hydrophilic fibers can further improve prevention of liquid component leakage during use even if the layered body is infused with a large amount of liquid components.

As for the fibers that constitute the non-extra-fine fibers, a mixture (fiber blend) containing hydrophilic fibers and hydrophobic fibers is more preferable because of its excellent balance between liquid retention and liquid release properties. The ratio (mass ratio) of the hydrophilic fibers relative to the hydrophobic fibers (hydrophilic fibers /hydrophobic fibers) can be selected from a range of about 99/1 to 1/99, and, for example, about 90/10 to 10/90. Where the proportion of hydrophilic fibers is too small, the fiber layered body tends to have difficulty for perviousness (compatibility) with the liquid components, so that there may be biased spots of the liquid amount in the fiber layered body, and the liquid retention property of the fiber layered body may tend to decrease. On the other hand, where the proportion of hydrophilic fibers is too large, the liquid retention capacity of the layered body may become too high, so that the layered body may have a difficulty to release the liquid components to the skin side during use.

Hydrophilic fibers are not limited to specific one as long as they have hydrophilic properties, and can be selected from synthetic fibers, natural fibers, and regenerated fibers that are produced from once-dissolved natural plant fibers or animal protein fibers into regenerated fibers by chemical treatment. Further, hydrophilic fibers are sufficient to at least have a surface composed of a hydrophilic resin. For example, hydrophilic fibers may include a fiber having a hydrophilically-treated surface, or a composite fiber comprising a hydrophobic resin as an inner (core) portion of the composite fiber.

Examples of synthetic fibers may include fibers of resins having hydrophilic groups such as hydroxyl groups, carboxyl groups, and sulfonic acid groups, particularly hydroxyl groups, in their molecules, for example, polyvinyl alcohol-based resins, polyamide-based resins, polyester-based resins such as polylactic acid, and (meth)acrylic copolymeric resins containing (meth)acrylamide units. These synthetic fibers can be used singly or in combination of two or more. Among these synthetic fibers, fibers of a hydrophilic resin having a hydroxyl group in the monomer unit are preferable, and ethylene-vinyl alcohol copolymer fibers are particularly preferable due to having hydroxyl groups uniformly in the molecules.

The ethylene-vinyl alcohol copolymer may have an ethylene unit content (copolymerization ratio) of, for example, about 10 to 60 mol%, preferably about 20 to 55 mol%, and more preferably about 30 to 50 mol%. The saponification degree of the ethylene-vinyl alcohol copolymer is, for example, about 90 to 99.99 mol%, preferably about 95 to 99.98 mol%, and more preferably about 96 to 99.97 mol%. The viscosity average degree of polymerization is, for example, about 200 to 2500, preferably about 300 to 2000, and more preferably about 400 to 1500. Where a resin exhibiting adhesiveness under moist heat, such as an ethylene-vinyl alcohol copolymer, is used, a bulky and stable non-extra-fine fiber layer can be formed by the steam-jet process.

Examples of natural fibers may include cotton, silk, hemp, silk, and wool. These natural fibers can be used singly or in combination of two or more types. Of these, cotton and the like are widely used.

Examples of regenerated fibers may include rayon fibers such as viscose rayon fibers, and cellulosic fibers such as acetate fibers, lyocell fibers, cupra fibers, and polynosic fibers. These regenerated fibers can be used singly or in combination of two or more types. Of these, rayon fibers and lyocell fibers are widely used.

As for a fiber having a surface composed of hydrophilic resin, the method for imparting hydrophilicity to the surface of the fiber may include a method in which a fiber-formable resin and a hydrophilic resin are spun together to obtain a composite fiber having a surface of at least a part thereof covered with the hydrophilic resin. The composite fibers formed by the method of covering the surface of the fiber with the hydrophilic resin are preferable because the hydrophilic performance of the fiber is less degraded even after prolonged use. Further advantage of the method of spinning a fiber-formable resin and a hydrophilic resin is to shorten the manufacturing time as well as to provide homogeneous and high hydrophilicity. In particular, from the viewpoint of high hydrophilicity, a fiber in which the entire surface of the fiber is covered with a hydrophilic resin in a sheath shape, that is, a composite fiber having a core-sheath structure in which the sheath portion is made of a hydrophilic resin is preferable.

Although core-sheath type composite fibers are not particularly limited as long as the sheath portion is composed of hydrophilic resin, the core portion is preferably composed of a hydrophobic resin, which constitutes hydrophobic fibers described below, because the core portion retains the fiber shape even when infused with liquid components so as to make it possible to suppress degradation of hydrophilic performance. Further, among hydrophobic resins, for example, polypropylene-based resins, polyester-based resins, and polyamide-based resins are preferred, and especially polyester-based resins such as a polyethylene terephthalate are preferred from the viewpoint of their excellent balance of heat resistance and fiber formability. It should be noted that the hydrophilic resin in the sheath portion is preferably a resin constituting a synthetic fiber, especially a polyvinyl alcohol-based resin such as an ethylene-vinyl alcohol copolymer, from the viewpoint of being able to produce bulky and stable nonwoven fabrics. The core-sheath type composite fiber may have a sheath/core ratio (mass ratio) of the sheath portion relative to the core portion of, for example, about 90/10 to 10/90 (for example, about 60/40 to 10/90), preferably about 80/20 to 15/85, and even more preferably about 60/40 to 20/80.

Among these hydrophilic fibers, cellulosic fibers, such as rayon fibers and lyocell fibers, are particularly preferred because of their high liquid absorbance and retention performances because these fibers can make water, aqueous solutions, polar solvents, and their emulsions constituting liquid components such as cosmetics to penetrate into inner side of the fibers. Alternatively, although ethylene-vinyl alcohol copolymer fibers (in particular, core-sheath type composite fibers whose sheaths are composed of ethylene-vinyl alcohol copolymer) are less effective than cellulosic fibers in liquid-retaining performance, the ethylene-vinyl alcohol copolymer fibers have good perviousness (compatibility) with liquid components such as cosmetics, and further have good liquid-releasability by pressure because the fibers themselves do not absorb liquid components. Therefore, cellulosic fibers and ethylene-vinyl alcohol copolymer fibers may be selected depending on the viscosity and amount of liquid components such as cosmetics, and may be combined in order to control the liquid retention and release properties. Furthermore, other fibers may be blended as needed.

Hydrophobic fibers or non-hydrophilic resins (resins with relatively high hydrophobicity with rather small polarity) constituting the non-extra-fine fiber layer may be used to achieve stability in shape of the non-extra-fine fiber layer.

Hydrophobic fibers provide a property for maintaining the bulk and firmness of the non-extra-fine fiber layer, since there is almost no decrease in Young's modulus of the hydrophobic fibers even when the non-extra-fine fiber layer is wet.

Such hydrophobic fibers are not particularly limited to specific one, and may include fibers comprising a resin with an official moisture content of less than 2.0% under a standard condition (20°C, 65% RH), for example, polyolefinic resins such as polyethylene and polypropylene; polyester-based resins such as polyethylene terephthalate, polybutylene terephthalate, and polyethylene naphthalate; polyacrylonitrile-based resins; and resins generally used for nonwoven fabrics. These hydrophobic fibers can be used singly or in combination of two or more types. Of these, polyester fibers are preferred due to their high versatility and excellent mechanical properties.

The cross-sectional shapes of the fibers (hydrophilic fibers as well as hydrophobic fibers) constituting the non-extra-fine fiber layer are not specifically limited, and may be of various shapes, for example, with round cross-section, irregular cross-section (flattened, elliptical cross-section, etc.), polygonal cross-section, multi-leafed cross-section (3-leafed to 14-leafed cross-section, etc.), hollow cross-section, V-shaped cross-section, T-shaped cross-section, H-shaped cross section, I-shaped (dog bone) cross-section, array-shaped cross-section, and others. Of these, round cross-section shapes and elliptical cross-section shapes are preferred.

The basis weight of the non-extra-fine fiber layer is, for example, about 20 to 200 g/m$^2$, preferably about 25 to 150 g/m$^2$, and even more preferably about 30 to 120 g/m$^2$ (especially about 30 to 100 g/m$^2$). Where the basis weight of the non-extra-fine fiber layer is too small, the liquid retention property as well as mechanical strength of the resulting fiber layered body tend to decrease. Where the basis weight is too large, the liquid component tends to be absorbed into the non-extra-fine fibers in large quantities, so that there is a difficulty for the liquid components to reach the skin.

The thickness of the non-extra-fine fiber layer can be selected from a range of about 100 to 3000 $\mu$m, for example, about 200 to 2000 $\mu$m, preferably about 300 to 1500 $\mu$m, and even more preferably about 400 to 1200 $\mu$m (especially about 400 to 1000 $\mu$m).

From the viewpoint of ensuring liquid retention and the strength of the fiber layered body, the density of the non-extra-fine fiber layer can be selected from a range of about 0.05 to 0.25 g/cm$^3$, for example, 0.08 to 0.20 g/cm$^3$, preferably 0.10 to 0.18 g/cm$^3$, and even more preferably 0.12 to 0.15 g/cm$^3$ (in particular, about 0.13 to 0.15 g/cm$^3$).

Extra-fine fiber layer

The extra-fine fiber layer used in the present invention comprises thermoplastic elastomeric fibers with an average fiber diameter of less than 10 $\mu$m. By using thermoplastic elastomeric fibers with elasticity and integrating the extra-fine fiber layer of the thermoplastic elastomeric fibers with the non-extra-fine fiber layer, the resulting fiber layered body also achieves excellent elasticity. The number average fiber diameter of single fibers in the thermoplastic elastomeric fibers is preferably 9 $\mu$m or less, and more preferably 8 $\mu$m or less. Where the average fiber diameter of the thermoplastic elastomeric fibers is 10 $\mu$m or more, it may be difficult for the thermoplastic elastomeric fibers to enter into the non-extra-fine fiber layer. Moreover, since the thermoplastic elastomeric fibers with the average fiber diameter of 10 $\mu$m or more may have difficulty to deform in conformity to the form of the non-extra-fine fiber layer at the interface between the extra-fine fiber layer and the non-extra-fine fiber layer, it is disadvantageous for the fiber layered body to improve the interlayer peel strength. In addition, when the extra-fine fiber layer comes into contact with the skin, the fiber layered body has insufficient adhesion to the skin.

Thermoplastic elastomeric fibers are not particularly limited to specific one, but are, for example, fibers made of a resin containing at least 30 mass% of thermoplastic elastomer, preferably fibers made of a resin containing at least 50 mass% of thermoplastic elastomer, more preferably fibers made of a resin containing at least 80 mass% of thermoplastic elastomer, and even more preferably fibers consisting of thermoplastic elastomeric resin.

Thermoplastic elastomeric fibers include polyurethane-based elastomeric fibers, polystyrene-based elastomeric fibers, polyolefinic elastomeric fibers, polyester-based elastomeric fibers, polyvinyl chloride-based elastomeric fibers, polyamide-based elastomeric fibers, and other elastomeric fibers. In view of improvement in liquid retention and elasticity, polyurethane-based elastomeric fibers and polystyrene-based elastomeric fibers, especially polyurethane-based elastomeric fibers are preferred.

The polyurethane-based elastomer that constitutes the polyurethane-based elastomeric fibers comprises hard segments composed of low molecular glycols and diisocyanates, and soft segments composed of high molecular diols and diisocyanates.

Examples of the low molecular glycols may include C$_{1-10}$diols such as ethylene glycol, 1,4-butanediol, and 1,6-hexanediol. Examples of the high molecular diols may include poly(l,4-butylene adipate), poly(1,6-hexane adipate), polycaprolactone, polyethylene glycol, polypropylene glycol, polyoxytetramethylene glycol, etc. Examples of the diisocyanates may include tolylene diisocyanate, 4,4-diphenylmethane diisocyanate, hexamethylene diisocyanate, and isophorone diisocyanate.

The polystyrene-based elastomers constituting polystyrene-based elastomeric fibers include SBS (styrene/butadiene/styrene block copolymer), SIS (styrene/isoprene/styrene block copolymer), SEBS (styrene/ethylene/butadiene/styrene block copolymer), SEPS (styrene/ethylene/propylene/styrene block copolymer), etc.

The olefinic elastomer constituting the polyolefinic elastomeric fibers may comprise polyethylene or polypropylene as hard segments and SEBS or ethylene/propylene copolymer as soft segments.

The polyester-based elastomers constituting polyester-based elastomeric fibers may comprise saturated polyester as hard segments and aliphatic polyether or aliphatic polyester as soft segments.

The polyvinyl chloride-based elastomers constituting polyvinyl chloride-based elastomeric fibers may comprise crystalline polyvinyl chloride as hard segments and amorphous polyvinyl chloride or acrylonitrile as soft segments.

The polyamide-based elastomers constituting polyamide-based elastomeric fibers may comprise polyamide as hard segments, and polyether or polyester, which are amorphous and have a low glass transition temperature, as soft segments.

The basis weight of the extra-fine fiber layer may be, for example, 50 g/m$^2$ or less, preferably 20 g/m$^2$ or less, more preferably 3 to 20 g/m$^2$, and even more preferably 5 to 20 g/m$^2$. Where the basis weight of the extra-fine fiber layer is too small, the lifting or tightening effect tends to be reduced due to decreased elasticity. On the other hand, where the basis weight of the extra-fine fiber layer is too large, the release of liquid components from the fiber layered body tends to be reduced.

The thickness of the extra-fine fiber layer can be selected from a range of 10 to 500 $\mu$m, for example, 30 to 500 $\mu$m, preferably 30 to 200 $\mu$m, and even more preferably 35 to 150 $\mu$m (especially 40 to 100 $\mu$m). If the thickness is too small, the amount of fibers to form an extra-fine fiber layer tends to be insufficient, making it difficult to form a uniform extra-fine fiber layer on the skin surface. On the other hand, too large thickness may lead to difficulty in permeation of liquid components into the extra-fine fiber layer from the non-extra-fine fiber layer.

From the viewpoint of ensuring the release of the liquid component as well as improving the elasticity (in particular, stretchability and adherence to the skin), the density of the extra-fine fiber layer can be selected from a range of about 0.10 to 0.40 g/cm$^3$, for example, 0.12 to 0.35 g/cm$^3$, preferably 0.15 to 0.30 g/cm$^3$, and even more preferably 0.18 to 0.28 g/cm$^3$ (especially 0.20 to 0.25 g/cm$^3$).

The extra-fine fiber layer may be an extra-fine fiber layer obtained by spraying thermoplastic elastomeric fibers in a molten state onto the non-extra-fine fiber layer (direct-blowing method), from the viewpoint of easier production of an extra-fine fiber layer with an average fiber diameter in the above range as well as enhanced integration with the non-extra-fine fiber layer. Here, the direct-blown nonwoven fabric may also be referred to as a melt-blown nonwoven fabric.

Producing Method of Fiber Layered Body

The process for disposing an extra-fine fiber layer on a non-extra-fine fiber layer comprises blowing thermoplastic elastomeric fibers in a molten state onto the non-extra-fine fiber layer (direct blowing method) to form the extra-fine fiber layer in order to make the process easier and to minimize post-processing effect on each fiber layer.

In more detail, in the process of forming an extra-fine fiber layer, thermoplastic elastomeric fibers in a deformable state are blown onto a non-extra-fine-fiber sheet to be a non-extra-fine fiber layer. When the thermoplastic elastomeric fibers are in contact with the fiber aggregate that constitutes the non-extra-fine-fiber sheet, some of the deformable thermoplastic elastomer fibers can enter into inner side of the fiber aggregate thanks to their deformability and momentum of being blown. Furthermore, even when the deformable thermoplastic elastomeric fibers in contact with the non-extra-fine fiber layer fail to enter into the inner side of the non-extra-fine fiber layer, some of the thermoplastic elastomer fibers can deform in conformity to fiber shapes of the non-extra-fine fiber layer surface at the interface with the non-extra-fine fiber layer.

The meltblowing method is a suitable method for blowing the thermoplastic elastomeric fibers in the molten state. By collecting the thermoplastic elastomeric fibers on the non-extra-fine fiber layer before they are completely solidified by the meltblowing method, a fiber layered body can be continuously produced with sufficient adhesion between the layers.

To the extent that the fiber layered bodies according to the present invention can be obtained, other disposing methods may be combined in addition to the aforementioned direct-blowing method. For example, other disposing methods may include air through method, steam-jet method, calendaring method, and spunlacing method.

In order for the resulting fiber layered body to have a peeling strength of 0.40 N/5 cm or higher, it is particularly important to adjust the spinning temperature and the collection distance, i.e., a distance from the spinning position of the thermoplastic elastomeric fibers in the molten state to the non-extra-fine fibers. The suitable collection distance to achieve the effect of the present invention depends on the spinning conditions such as spinning temperature and discharged amount, the type of elastomeric resin used, the fiber diameters of the extra-fine fibers, the environmental temperature, and others.

For example, where a polyurethane-based elastomeric resin is used as the material for the fibers constituting the extra-fine fiber layer, the spinning temperature in the meltblowing method is preferably about 240 to 270°C, more preferably about 240 to 265°C, and even more preferably about 240 to 260°C.

Where a polystyrene-based elastomeric resin is used as the material for the fibers constituting the extra-fine fiber layer, the spinning temperature of the meltblowing method is preferably about 200 to 350°C, more preferably about 220 to 320°C, and even more preferably about 240 to 300°C.

The collection distance, which is the distance from the spinning position of the thermoplastic elastomeric fibers in the molten state to the non-extra-fine fibers during the meltblowing method, can be changed as appropriate depending on the type of resin constituting the thermoplastic elastomeric fibers and the spinning temperature, and is preferably about 8 to 20 cm, more preferably about 10 to 18 cm, and even more preferably about 10 to 15 cm. This collection distance is shorter than the typical collection distance employed in meltblowing methods.

By performing direct blowing method in which discharge from the spinning nozzle is carried out directly to the non-extra-fine fiber layer at a shorter collection distance than usual, the thermoplastic elastomer in the molten state that is discharged from the spinning nozzle onto the non-extra-fine fiber layer can utilize the momentum of the discharge to allow some of the thermoplastic elastomeric fibers to physically enter into the non-extra-fine fiber layer. Furthermore, on the contact surface (or interface) with the non-extra-fine fiber layer, since the thermoplastic elastomer in the form of soft (deformable) extra-fine fibers brings into contact with the non-extra-fine fiber layer, it allows the thermoplastic elastomer to be deformed to fit the shape of the non-extra-fine fibers. As a result, the peel resistance strength between the extra-fine fiber layer and the non-extra-fine fiber layer can be improved.

On this point, in general, where the collection distance is too close, although the adhesive strength improves, due to the small fiber diameter of the extra-fine fiber layer, the heat tends to fuse the thermoplastic elastomer fibers together to form a film, resulting in reduction of liquid component release as well as of adhesion to skin. Where the collection distance is too far apart, the thermoplastic elastomeric fibers are cooled before their collection, which tends to solidification of the fibers, so that it may be difficult to achieve sufficient peeling strength.

The spacing of the spinning holes in the meltblowing method is, for example, about 100 to 4000 holes/m, preferably about 500 to 3000 holes/m, and even more preferably about 1000 to 2500 holes/m. The discharge amount from single hole is, for example, about 0.01 to 1 g/hole/min, preferably 0.05 to 0.5 g/hole/min, and even more preferably 0.1 to 0.3 g/hole/min.

The air pressure of the high-temperature air in the meltblowing method can be selected from a range of about 0.01 to 1 MPa, and may be for example about 0.05 to 0.8 MPa, preferably about 0.1 to 0.6 MPa, more preferably about 0.2 to 0.5 MPa. The air temperature is for example a temperature near the spinning temperature, for example, 0 to 50°C higher than the spinning temperature, preferably 3 to 30°C higher than the spinning temperature, and even more preferably 5 to 20°C higher than the spinning temperature.

The conveyor speed in the meltblowing method is, for example, about 1 to 200 m/min, preferably about 5 to 100 m/min, and even more preferably about 10 to 80 m/min. By adjusting the air pressure, the conveyor speed, and the distance (collection distance) between the nozzle tip and the conveyor (such as a net conveyor) as appropriate, the basis weight, density, and flexibility of the resulting adhesion layer (extra-fine fiber layer) may be adjusted.

Physical Properties of Fiber Layered Body

The fiber layered body has a peeling strength between layers (delamination strength) of 0.40 N/5cm or higher, and preferably 0.50 N/5cm or higher. Where the peeling strength is lower than 0.40 N/5cm, the fiber layered body will be delaminated upon stretching before use. Moreover, delamination of the extra-fine fiber layer from the non-extra-fine fiber layer during use may prevent satisfactory release of the liquid components to the skin. The upper limit of the peeling strength is not particularly limited, but for the fiber layers deposed by a general method, there is a possibility that one of the fiber layers is destroyed if the peeling strength exceeds 5N/5cm. In such a case, accurate numerical measurement cannot be performed. Accordingly, the upper limit of the peeling strength is preferably 5 N/5cm or lower.

It is preferable that surface unevenness of the fiber layered body is small. More specifically, it is important that the ratio of the unevenness relative to the total thickness of the fiber layered body is 40% or less, preferably 35% or less, and more preferably 30% or less. Where the ratio of the unevenness exceeds 40%, the adhesion to the skin is reduced due to fewer adhesion spots to the skin, so that the satisfactory lifting effect cannot be achieved. It is preferred that the ratio of the unevenness is lower, and the lower limit is not particularly limited, and it may be, for example, about 1% or more, preferably about 3% or more, more preferably about 5% or more, and still more preferably about 10% or more.

The thickness of the fiber layered body can be selected from a range of about 0.1 to 4 mm, for example, 0.15 to 3 mm, preferably 0.2 to 2 mm, more preferably 0.25 to 1.5 mm (particularly 0.3 to 1 mm). Where the thickness is too small, the liquid retention property tends to decrease. On the other hand, where the thickness becomes too large, the amount of liquid retained in the fiber layered body (product) becomes too large, which tends to make the product heavier and reduce the adhesion to the skin.

The basis weight of the fiber layered body is, for example, about 21 to 250 g/m$^2$, preferably, about 25 to 200 g/m$^2$, and more preferably, about 30 to 150 g/m$^2$ (particularly, about 30 to 130 g/m$^2$).

The breaking strength (strength at break) of the fiber layered body may be, for example, about 120 to 220 N/5cm, preferably about 130 to 200 N/5cm, and more preferably about 140 to 190 N/5cm in the longitudinal direction (MD: machine direction) during the production of the fiber layered body. Further, the breaking strength of the fiber layered body may be, for example, about 18 to 45 N/5cm, preferably about 19 to 40 N/5cm, and more preferably about 20 to 38 N/5cm in the lateral direction (CD: cross direction) during the production of the fiber layered body.

The breaking elongation (elongation at break) of the fiber layered body may be, for example, about 15 to 40%, preferably about 18 to 35%, and more preferably about 20 to 33% in the longitudinal direction (MD: machine direction) during the production of the fiber layered body. Further, in the lateral direction (CD: cross direction) during the production of the fiber layered body, the breaking elongation thereof may be, for example about 140 to 180%, preferably about 143 to 175%, more preferably about 145 to 170%.

The water retention ratio of the fiber layered body of the present invention is preferably about 700 to 1500 mass%, more preferably about 700 to 1300 mass%, and still more preferably about 710 to 1000 mass%. Where the water retention ratio is too low, there is a tendency that the fiber layered body may retain liquid component insufficiently so as to cause leaking, and that available time of the product becomes too short to exert satisfactory effects. On the other hand, too high water retention ratio of the fiber layered body tends to decrease property to release liquid components.

The fiber layered body according to the present invention is excellent in adhesion to the skin. For example, the fiber layered body may have a friction force (adhesion) of, for example, about 0.5 N to 3.0 N, preferably about 0.6 N to 2.7 N, more preferably about 0.7 N to 2.5 N, and even more preferably about 0.8 N to 2.0 N that is measured, with reference to ASTM-D1894, by subjecting the fiber layered body to be impregnated with a cosmetic (lotion) ("Freshel Essence Lotion NA" manufactured by Kanebo Cosmetics Inc.) at a proportion of 700 mass% of the sheet mass.

Further, the fiber layered body according to the present invention still has excellent adhesion to the skin even when the amount of infused liquid is extremely small, and can maintain the integrity between layers. For example, the friction force (adhesion) may be, for example, about 1.0 N to 4.0 N, preferably about 1.1 N to 3.7 N, more preferably about 1.2 N to 3.5 N measured, with reference to ASTM-D1894, by subjecting the fiber layered body to be infused with a cosmetic (lotion) ("Freshel Essence Lotion NA" manufactured by Kanebo Cosmetics Inc.) at a proportion of 300 mass% of the sheet mass.

At the time of the aforementioned 300 mass% infusion, there is a tendency that delamination between the extra-fine fiber layer and the non-extra-fine fiber layer would occur, but the fiber layered body according to the present invention has an excellent integrated structure between the extra-fine fiber layer and the non-extra-fine fiber layer, and thus preferably does not cause delamination between the layers upon the aforementioned friction test.

The fiber layered body has a recovery ratio of preferably about 60% or higher, more preferably about 62% or higher, and still more preferably about 63% or higher after 25% elongation under wet. Too low elongation recovery ratio may cause insufficient lifting effect. The upper limit of the elongation recovery ratio is not particularly limited, and it is preferable to be as high as possible. For example, the elongation recovery ratio is about 95% or less, and may be about 90% or less.

Use of Fiber Layered Body

The fiber layered body according to the present invention can be used in various applications where elasticity of the thermoplastic elastomer fibers is applicable, and may be used in a dry state without additional infusion process. For example, in applications where a dry fiber layered body is used to absorb liquid components, the fiber layered body according to the present invention can be used for application for human usage as sheets for absorbing body fluids (or sheets for washing the skin), for example, surface materials for sanitary goods such as napkins and diapers, diaper liners, sweat absorbing sheets (for example, sweat absorbable pads, especially underarm sweat pads) and others, etc. As non-application for human usage, the fiber layered body can be used as a liquid-absorbable sheet or a liquid absorbent mat, such as a condensation water absorption sheet.

Alternatively, a liquid component(s) may be applied to the fiber layered body according to the present invention so that the fiber layered body can be used as a liquid-infused sheet in a wet state. Although the liquid-infused sheet can be used for general purpose applications such as wet wipes (wet tissue paper), since the fiber layered body according to the present invention is excellent in liquid retention property in addition to its adhesion and fit to the skin, the fiber layered body can be preferably used for application to human usage as a liquid infused sheet in which the fiber layered body is infused with liquid components such as beauty ingredients and medicinal ingredients, to adhere to skin, for example, various skin care sheets such as face masks, make-up removal sheets or cleansing sheets, body refreshing sheets (sweat wiping sheets, oil absorbing sheets, etc.), cooling sheets, and medicinal or therapeutic sheets (anti-itch sheets, adhesive bandages, topical patches, etc.). Further, as non-application for human usage, the fiber layered body can be also suitably used for applications other than the human body, for example, water- or chemical-infused wiping sheets for properties, agricultural sheets, biological medium pads, and others.

EXAMPLES

Hereinafter, the present invention will be described based on Examples, but the present invention is not limited to the present Examples. In the following Examples and Comparative Examples, various physical properties were measured by the following methods.

Average Fiber Diameter of Fibers

Fiber structures were observed using a scanning electron microscope. From an electron micrograph, 100 fibers were randomly selected and diameters thereof were measured to determine the number-average fiber diameter of the single fibers. Thus-obtained number-average fiber diameter was used as the average fiber diameter of the fibers.

Basis Weight (g/m²)

With reference to JIS L 1913, a sample was left to a standard condition at a temperature of 20°C and under a humidity of 65% for 24 hours. Thereafter, a specimen with 30 cm in width and 30 cm in length was obtained from the sample and weighed using a pair of balances to measure a weight (g). The obtained weight was converted into a weight per 1 m × 1 m and used as the basis weight.

Thickness ($\mu$m)

With reference to JIS L 1913, a load of 12 g/cm² was applied to a sample to measure a thickness using a thickness measuring device. The measured result was used as the thickness.

Breaking Strength / Elongation

Both strength and elongation at break were measured using a precision universal testing machine ("Autograph AGS-D type" manufactured by Shimadzu Corporation) with reference to JIS L 1913. That is, were collected test pieces each having a width of 50 mm and a length of 200 mm, both in the longitudinal direction (MD: machine direction) of manufacturing the fiber layered body and in the lateral direction (CD: cross direction of the machine direction). The width of the gripping parts for each test piece was set to 10 cm. After opposed parts of the test piece were fixed at the gripping parts, the gripping parts were pulled at a speed of 300 mm/min until breakage of the test piece occurred, and the test force was measured as breaking strength. The average value of the test forces at the time of test piece breakage was used as the breaking strength. Moreover, the elongated distance at breakage of the test piece was divided by the width of the gripping area (10 cm) and expressed as a percentage, which was evaluated as the breaking elongation.

Peeling Strength

The peeling strength of a test piece was measured using a precision universal testing machine ("Autograph AGS-D type" manufactured by Shimadzu Corporation) with reference to JIS L 1085. That is, by making a kraft tape adhered to the extra-fine fiber layer side of the test piece having a width of 50 mm and a length of 200 mm with respect to the longitudinal direction of manufacturing the nonwoven fabric, the extra-fine fiber layer and the non-extra-fine fiber layer are peeled off at an end portion of the test piece. After setting the width of the gripping parts as 5 cm at each of the peeled ends, the gripping parts were fixed and peeled off at a speed of 200 mm/min from each other. The average value of the test forces required for peeling was determined as the peeling strength.

Water Retention Ratio

Water retention ratio of the fiber layered body was measured with reference to JIS L 1913.6.9.2. That is, a test piece (5 cm × 5 cm) is collected from a fiber layer body as a sample, and the test piece is precisely weighed. After submerging the test piece in water for 30 seconds, the test piece was gently taken out of the water. After leaving the water-dripping test piece as it was for 1 minute, the test piece was precisely weighed again, and the difference in mass before and after immersion in water was used as the water retention amount. The water retention amount was divided by the mass of the test piece before immersion in water, and the obtained value was expressed in percentage as the water retention ratio.

Adhesion

The friction force was measured with reference to ASTM-D1894 using a precision universal testing machine ("Autograph AGS-D type" manufactured by Shimadzu Corporation). A sample of the fiber layered body was cut out to 4.0 cm in the MD (longitudinal direction, machine direction when manufacturing the fiber layered body) and to 11.0 cm in the CD direction (width direction when manufacturing the fiber layered body) with a gripping part of 1 cm and a ground part of 10 cm. Further, assuming a face mask, the sample infused with cosmetics was taken out and pushed with the fingertips of both hands so as to spread the infused cosmetics evenly in the sample, and then two types of samples were prepared with two different mass percentages of cosmetics as shown below. A test was conducted to pull the gripping part of the sample infused with a specific amount of cosmetics in the direction grasped with a clip. In detail, an acrylic plate was fixed on the table for measuring the friction force, and the sample was placed in the center portion of the plate in which the surface of the sample applicable to skin was placed toward downward in contact to the plate. Further, after protecting the sample with a PE film, a weight was placed on the sample through the PE film so as to apply an even load of 10 g/cm$^2$ to a portion of MD 4.0 cm × CD 10.0 cm of the sample for 10 seconds, and then the weight and the PE film were removed from the sample so as to attain close contact of the sample with the acrylic plate. Then, using a testing machine equipped with a load cell, the friction force was measured by horizontally pulling the polyamide yarn tied to the clip via a pulley at a speed of 20 mm/min. The peak value of the test force was defined as the adhesion. Adhesion was measured under the following two conditions:

(1) Infusion of 300 mass% of cosmetics with respect to the sample mass, imitating the environment in the latter half period of the use of the face mask. In such a condition, adhesion of each of the samples was determined.
(2) Infusion of 700 mass% of cosmetics with respect to the sample mass, imitating the environment soon after the use of the face mask is started. In such a condition, adhesion of each of the samples was obtained.

The cosmetics infusion was carried out with aqueous cosmetics lotion containing water and hydrophilic materials as its main ingredients. That is a composition containing water, glycerin, ethanol, dipropylene glycol, maltitol, PEG-75, raffinose, phenyl trimethicone, carbomer K, polysorbate 20, perfluoroalkyl dimethicone polyol, 1,3-butylene glycol, cucumber extract, PEG-60 hydrogenated castor oil, xanthan gum, aloe vela extract-1, edetate, phenoxyethanol, and paraben as ingredients.

Surface Unevenness of the Fiber Layered Body Relative to Thickness

Using a razor blade ("Feather Razor Blade S Single-edged", manufactured by Feather Safety Razor Co., Ltd.), an obtained fiber layered body was cut to give a sample in which the sample was cut in the direction perpendicular to the surface thereof and at 45 degrees to the MD. The sample was photographed at ten points in the cross-section using a digital microscope [VHX-900 Digital Microscope manufactured by KEYENCE CORPORATION] at a magnification of 100 times. At the time of photographing each of the images, each of the view fields was set such that the cross section continues sequentially in the lateral direction. After measuring the thickness of the thickest and thinnest portions in each image, the average values of the thickest and thinnest portions among the images were calculated, and the average values were designated as TA and TB, respectively. Then, the difference in thickness between the thickest and thinnest portions was divided by the thickness of the thickest portion using the following equation (1), and the resulting percentage value was used as surface unevenness of the fiber layered body relative to the thickness of the fiber layered body.

$$(TA-TB) / TA \times 100 \qquad (1)$$

TA: the average of the thickness of the thickest portions.
TB: the average of the thickness of the thinnest portion.

Recovery Ratio After 25% Elongation Under Wet

The recovery ratio after 25% elongation under wet was measured in accordance with the method described in JIS L 1913 (General short fiber nonwoven fabric) 6.3.2 (Tensile strength and elongation ratio test under wet). Specifically, the samples were placed in water at 20°C ± 2 °C until they settled down due to their own weight, or submerged in water for more than 1 hour, then the samples were taken out from the placed water to immediately measure a recovery ratio of the sample after 25% elongation under wet.
The following materials were prepared as raw materials for the fiber layered body.

Rayon fibers: Regenerated cellulose fibers, "Hope" manufactured by Omikenshi Co., Ltd., average fiber diameter of 12.6 μm, fiber length of 40 mm.

Polyester fibers: Polyethylene terephthalate (PET) fibers manufactured by Toray Industries, Inc., average fiber diameter of 12.8 μm, fiber length of 51 mm.

Polyurethane resin (PU): Melt viscosity of $1.0 \times 10^3$ to $5.0 \times 10^3$ Pa·s/200°C when measured in accordance with D-65935 constant temperature method.

Styrenic thermoplastic elastomer: "EARNESTON (registered trademark)" CJ101 manufactured by Kuraray Plastics Co., Ltd.

Polypropylene resin (PP): MFR (230°C, 2.16 kg) = 1100 g/10 min.

Preparation of Non-Extra-fine fiber layer

Rayon fibers (40 parts by mass) were uniformly blended with polyester fibers (60 parts by mass), then blended fibers were subjected to a conventional method to obtain a semi-random-card web with a basis weight of 55 g/m². The card web was placed on a punching drum support having an aperture ratio of 25% and a hole diameter of 0.3 mm and continuously conveyed in a longitudinal direction at a speed of 50 m/min, while high pressure water jets were injected from above onto the semi-random card web for hydroentanglement process so as to obtain an entangled fiber web (nonwoven fabric). In the entanglement process, 2 nozzles were used at a distance between the adjacent nozzles of 20 cm, and each of the nozzles had orifices with a hole diameter of 0.10 mm at intervals of 0.6 mm in a crosswise direction of the web. The high-pressure water jets injected from the first nozzle had a water pressure of 3.0 MPa, and the high-pressure water jets injected from the second nozzle had a water pressure of 4.0 MPa as hydroentanglement process. Further hydroentanglement process was performed using 2 nozzles each of which had orifices with a hole diameter of 0.10 mm at intervals of 0.6 mm in a crosswise direction of the web. Each of the high-pressure water jets injected from the 2 nozzles had a water pressure of 5 MPa. After hydroentanglement process, the web was dried at 120°C to obtain a spunlaced nonwoven fabric having a thickness of 0.40 mm and a basis weight of about 51 to 55 g/m² as a non-extra-fine fiber layer.

Example 1

Using a typical meltblowing equipment, a polyurethane resin was spun by meltblowing process at a spinning temperature of 243°C, an air temperature of 253°C, an air pressure of 0.4 MPa, a discharge rate from a single hole of 0.2 g/hole/minute, 400 spinning holes per spinneret (single row arrangement), with placing the non-extra-fine fiber layer on the rotating net conveyer to be passed through. The collection distance was set to 10 cm. In such a manner, an extra-fine fiber layer (basis weight of 5 g/m²) of polyurethane fibers having an average fiber diameter of 5.51 μm was disposed on a non-extra-fine fiber layer to obtain a fiber layered body having a thickness of 0.42 and a basis weight of 58.0 g/m², and then the fiber layered body was wound up.

The obtained fiber layered body had a peeling strength of 1.44 N/5cm and a recovery ratio of 63.4% after 25% elongation under wet, indicating that the fiber layered body was free from delamination during use and had a good lifting effect. Further, when the obtained fiber layered body was infused with cosmetics and the adhesion thereof was measured, the adhesion at 700 mass% infusion was as high as 0.89 N. Furthermore, the adhesion at 300 mass% infusion was 1.66 N, which was higher than that at 700 mass% infusion.

Example 2

A fiber layered body in which the average fiber diameter of the polyurethane fibers was 5.46 μm, having a thickness of 0.46 mm and a basis weight of 65.6 g/m² was obtained in the same manner as in Example 1 except that the basis weight of the extra-fine fiber layer was adjusted to 10 g/m². The obtained fiber layered body had a peeling strength of 1.06 N/5cm and a recovery ratio of 72.2% after 25% elongation under wet, indicating that the fiber layered body was free from delamination during use and had a good lifting effect. Further, when the obtained fiber layered body was infused with cosmetics to measure adhesion thereof, the adhesion at 700 mass% infusion was as high as 0.63 N, indicating that the adhesion was satisfactory. Furthermore, the adhesion at 300 mass% infusion was 1.29 N, which was higher than that at 700 mass% infusion.

Example 3

A fiber layered body in which the average fiber diameter of the polyurethan fibers was 4.93 μm, having a thickness of 0.45 mm and a basis weight of 65.2 g/m² was obtained in the same manner as in Example 2 except that the collection distance when producing the extra-fine fiber layer was set to 15 cm. The obtained fiber layered body had a peeling strength of 0.50 N/5cm and a recovery ratio of 75.6% after 25% elongation under wet, indicating that the fiber layered body was free from delamination during use and had a good lifting effect. Further, when the obtained fiber layered body was infused with cosmetics to measure adhesion thereof, the adhesion at 700 mass% infusion was as high as 0.67 N, indicating that the adhesion was satisfactory. Furthermore, the adhesion at 300 mass% infusion was 1.18 N, which was higher than that at 700 mass% infusion.

Example 4

A fiber layered body in which the average fiber diameter of the polyurethan fibers was 1.99 μm, having a thickness of 0.45 mm and a basis weight of 64.7 g/m² was obtained in the same manner as in Example 2 except that the spinning temperature when producing the extra-fine fiber layer was set to 260°C. The obtained fiber layered body had a peeling strength of 0.95 N/5cm and a recovery ratio of 73.6% after 25% elongation under wet, indicating that the fiber layered body was free from delamination during use and had a good lifting effect. Further, when the obtained fiber layered body was infused with cosmetics to measure adhesion thereof, the adhesion at 700 mass% infusion was as high as 1.09 N, indicating that the adhesion was fine. Furthermore, the adhesion at 300 mass% infusion was 1.37 N, which was higher than that at 700 mass% infusion.

Example 5

Using a typical meltblowing equipment, a styrene-based elastomer was spun by meltblowing process at a spinning temperature of 250°C, an air temperature of 260°C, an air pressure of 0.4 MPa, a discharge rate from a single hole of 0.2 g/hole/minute, 400 spinning holes per spinneret (single row arrangement), with placing the non-extra-fine fiber layer on the rotating net conveyer to be passed through. The collection distance was set to 10 cm. In such a manner, an extra-fine fiber layer (basis weight of 10 g/m²) of styrene-based elastomer fibers having an average fiber diameter of 6.61 μm was disposed on a non-extra-fine fiber layer to obtain a fiber layered body having a thickness of 0.46 mm and a basis weight of 61.2 g/m², and then the fiber layered body was wound up.
The obtained fiber layered body had a peeling strength of 0.50 N/5cm and a recovery ratio of 48.8% after 25% elongation under wet, indicating less satisfactory lifting effect than other Examples. However, the adhesion at 700 mass% infusion was as high as 1.00 N, indicating that the fiber layered body was free from delamination during use, and that the adhesion was extremely high. Further, the adhesion at 300 mass% infusion was 1.20 N, which was higher than that at 700 mass% infusion.

Comparative Example 1

Using a typical meltblowing equipment, 100 parts by mass of polypropylene resin (MFR = 1100 g/10 min) was spun by meltblowing process at a spinning temperature of 285°C, an air temperature of 275 °C, an air pressure of 0.4 MPa, a discharge rate from a single hole of 0.2 g/hole/minute, 400 spinning holes per spinneret (single row arrangement), and the spun product was collected on the rotating net conveyer, the net conveyer serving as a support medium. In such a manner, an extra-fine fiber layer composed of meltblown nonwoven fabric having a basis weight of 5 g/m² was produced and wound up.
Next, 40 parts by mass of rayon fibers were uniformly blended with 60 parts by mass of polyester fibers, then blended fibers were subjected to a conventional method to obtain a semi-random-card web with a basis weight of 60 g/m². The card web was placed on a punching drum support having an aperture ratio of 25% and a hole diameter of 0.3 mm and continuously conveyed in a longitudinal direction at a speed of 50 m/min, while high pressure water jets were injected from above onto the semi-random card web for hydroentanglement process so as to obtain a non-extra-fine fiber layer. In the entanglement process, 2 nozzles were used at a distance between the adjacent nozzles of 20 cm, and each of the nozzles had orifices with a hole diameter of 0.10 mm at intervals of 0.6 mm in a crosswise direction of the web. The high-pressure water jets injected from the first nozzle had a water pressure of 3.0 MPa, and the high-pressure water jets injected from the second nozzle had a water pressure of 4.0 MPa as hydroentanglement process. The extra-fine fiber layer with a basis weight of 5 g/m², which was produced previously, was unwound from the unwinding device, overlapped with the non-extra-fine fiber layer, and placed on an entirely flat support medium having a fine mesh. Such an overlapped body was continuously conveyed, while high pressure water jets were injected onto the overlapped body for hydroentanglement process. In such a manner, the fibers constituting these 2 nonwoven fabrics were entangled and integrated. This hydroentanglement process was performed using 2 nozzles each of which had orifices with a hole diameter of 0.10 mm at intervals of 0.6 mm in a crosswise direction of the web. Each of the high-pressure water jets injected from the 2 nozzles had a water pressure of 5 MPa. After hydroentanglement process, the web was dried at 130°C to obtain a fiber layered body having a thickness of 0.40 mm and a basis weight of 64.0 g/m².
The obtained fiber layered body had a peeling strength of 0.99 N/5cm, which was sufficient to provide a good delamination force. However, since thermoplastic elastomeric fibers were not used in the fiber layered body, the recovery ratio after 25% elongation under wet was as low as 38.8%, indicating that the lifting effect was insufficient. Further, the adhesion at 300 mass% infusion of cosmetics was also inferior to those of Examples.

Comparative Example 2

The extra-fine fiber layer and the non-extra-fine fiber layer which were produced by the method of Comparative Example 1 were layered by partial thermocompression using a thermal embossing roll with a crimp area of 3.3% to obtain a fiber layered body having a thickness of 0.42 mm and a basis weight of 57.8 g/m$^2$. The peeling strength of the obtained fiber layered body was as low as 0.22 N/5cm. In addition, the recovery ratio after 25% elongation under wet was 41.6%, indicating unsatisfactory lifting effect. Further, the layers of the fiber layered body were delaminated when measuring the adhesion at infusion of 300 mass% cosmetic solution. Further, the adhesion at 700 mass% infusion was also inferior to those of Examples because of larger surface unevenness caused by the embossing process. Further, since no additional layering process other than embossing process was carried out, the breaking strength, both in the longitudinal direction and in the crosswise direction, of the fiber layered body was less than half those of Examples.

Comparative Example 3

The extra-fine fiber layer was formed on the non-extra-fine fiber layer in the same manner as in Example 2 except that the collection distance when producing the extra-fine fiber layer was set to 25 cm. Further, partial thermocompression using a thermal embossing roll with a crimped area of 3.3% was carried out to obtain a fiber layered body in which the average fiber diameter of the polyurethan fibers was 5.44 $\mu$m, having a thickness of 0.38 mm and a basis weight of 66.3 g/m$^2$. The obtained fiber layered body had a peeling strength of 1.57 N/5cm, indicating that the layers were solidly adhered. However, the adhesive force was 0.37 N at infusion of 700 mass% cosmetic solution, indicating insufficient adhesion as well as a possibility of delamination of the layers. Further, the adhesion at 700 mass% infusion was also inferior to those of Examples due to enlarged unevenness caused by the embossing process. Further, even when direct blowing was carried out, the effect of the direct blowing could not be sufficiently exerted because the collection distance was too far apart. Even when embossing process was carried out, the breaking strength values, both in the longitudinal direction and in crosswise direction, of the fiber layered body were lower than those of Examples.

Comparative Example 4

The fiber layered body in which the average fiber diameter of the polyurethan fibers was 5.44 $\mu$m, having a thickness of 0.44 mm and a basis weight of 65.8 g/m$^2$ was obtained in the same manner as in Example 2 except that the collection distance when producing the extra-fine fiber layer was set to 25 cm. The obtained fiber layered body had a reduced peeling strength of 0.09 N/5cm, and as a result, the layers of the fiber layered body were delaminated when measuring the adhesion at infusion of 300 mass% cosmetic solution.

Comparative Example 5

Attempt to produce a fiber layered body in the same manner as in Example 1 was made, except that the collection distance when producing the extra-fine fiber layer was set to 5 cm. However, since the collection distance was too close, the extra-fine fibers were fused and turned into film, making it impossible to produce a fiber layered body having an extra-fine fiber layer. The obtained layered body was infused with cosmetics to measure adhesion thereof. Due to the film formation instead of extra-fine fiber layer, the adhesion at 700 mass% infusion was 0.18 N, and that at 300 mass% infusion was 0.08 N, both of which were low values, indicating the insufficient adhesion.

[Table 1]

| Item | Unit | Ex. 1 | | Ex. 2 | | Ex. 3 | | Ex. 4 | | Ex. 5 | |
|---|---|---|---|---|---|---|---|---|---|---|---|
| Resin of extra-fine fiber layer | | PU | | PU | | PU | | PU | | Styrenic TPE | |
| Spinning temperature | °C | 243 | | 243 | | 243 | | 260 | | 250 | |
| Collection distance | cm | 10 | | 10 | | 15 | | 10 | | 10 | |
| Basis weight of extra-fine fiber layer | g/m² | 5 | | 10 | | 10 | | 10 | | 10 | |
| Average fiber diameter of extra-fine fibers | μm | 5.51 | | 5.46 | | 4.93 | | 1.99 | | 6.61 | |
| Disposing method | | DB | | DB | | DB | | DB | | DB | |
| Physical properties and evaluation of fiber layered body | | | | | | | | | | | |
| Basis weight | g/m² | 58.0 | | 65.6 | | 65.2 | | 64.7 | | 61.2 | |
| Thickness | mm/sheet | 0.42 | | 0.46 | | 0.45 | | 0.45 | | 0.46 | |
| Peeling strength | N/5cm | 1.44 | | 1.06 | | 0.50 | | 0.95 | | 0.50 | |
| Unevenness | % | 32 | | 19 | | 27 | | 28 | | 27 | |
| Water retention ratio | % | 759 | | 735 | | 748 | | 819 | | 732 | |
| Breaking strength long./cross | N/5cm | 164.2 | 29.8 | 165.0 | 34.4 | 160.3 | 33.7 | 183.9 | 29.3 | 144.0 | 31.9 |
| Breaking elongation long./cross | % | 26.7 | 156.9 | 26.9 | 160.5 | 26.6 | 157.4 | 27.7 | 158.4 | 31.3 | 147.8 |
| Recovery ratio after 25% elongation under wet | % | 63.4 | | 72.2 | | 75.6 | | 73.6 | | 48.8 | |
| Adhesion (300 mass% infusion) | N | 1.66 | | 1.29 | | 1.18 | | 1.37 | | 1.20 | |
| Adhesion (700 mass% infusion) | N | 0.89 | | 0.63 | | 0.67 | | 1.09 | | 1.00 | |

PU: Polyurethane, DB: direct blowing.

| Item | Unit | Com. Ex. 1 | Com. Ex. 2 | Com. Ex. 3 | Com. Ex. 4 | Com. Ex. 5 |
|---|---|---|---|---|---|---|
| Resin of extra-fine fiber layer | | PP | PP | PU | PU | PU |
| Spinning temperature | °C | 285 | 285 | 243 | 243 | 243 |
| Collection distance | cm | 10 | 10 | 25 | 25 | 5 |
| Basis weight of extra-fine fiber layer | g/m² | 5 | 5 | 10 | 10 | 5 |
| Average fiber diameter of extra-fine fibers | μm | 2.42 | 2.42 | 5.44 | 5.44 | - |
| Disposing method | | HE | EB | DB + EB | DB | DB |

(continued)

| Physical properties and evaluation of fiber layered body | | | | | | | | | | | |
|---|---|---|---|---|---|---|---|---|---|---|---|
| Basis weight | | g/m$^2$ | 64.0 | | 57.8 | | 66.3 | | 65.8 60.0 | | | |
| Thickness | | mm/sheet | 0.40 | | 0.42 | | 0.38 | | 0.44 | | 0.48 | |
| Peeling strength | | N/5cm | 0.99 | | 0.22 | | 1.57 | | 0.09 | | 5.40 | |
| Unevenness | | % | 27 | | 78 | | 78 | | 8 | | 31 | |
| Water retention ratio | | % | 581 | | 652 | | 592 | | 744 | | 699 | |
| Breaking strength | long./cross | N/5cm | 164.9 | 33.0 | 77.2 | 8.0 | 105.7 | 16.1 | 170.9 | 31.7 | 177.0 | 39.5 |
| Breaking elongation | long./cross | % | 24.1 | 138.3 | 28.4 | 175.6 | 26.6 | 173.1 | 28.1 | 157.1 | 154.0 | 34.9 |
| Recovery ratio after 25% elongation under wet | | % | 38.8 | | 41.6 | | 69.1 | | 74.4 | | 65.2 | |
| Adhesion (300 mass% infusion) | | N | 0.99 | | Delami. (0.93) | | 1.28 | | Delami. (0.53) | | 0.08 | |
| Adhesion (700 mass% infusion) | | N | 0.70 | | 0.55 | | 0.37 | | 0.94 | | 0.18 | |
| PU: Polyurethane, PP: Polypropylene, DB: direct blowing, HE: hydroentanglement, EB: embossing, Delami.: delamination. | | | | | | | | | | | |

INDUSTRIAL APPLICABILITY

The fiber layered body according to the present invention has excellent liquid retention and peeling strength while keeping sufficient mechanical strength and elongation as a fiber layered body. In addition, the fiber layered body has excellent stretchability and adhesion, which lead to capability for lifting effect. Accordingly, the fiber layered body can be used for applications where liquid components are absorbed therein. For example, it can be suitably used for, surface materials for absorbing body fluids such as sanitary napkins and diapers, sheets for absorbing body fluids such as diaper liners, sheets for washing the skin such as wet wipes, and liquid infused sheets such as face masks.

**Claims**

1. A fiber layered body comprising:

    an extra-fine fiber layer which comprises thermoplastic elastomeric fibers and has an average fiber diameter of less than 10 μm; and
    a non-extra-fine fiber layer which comprises fibers having an average fiber diameter of 10 to 30 μm and is adjacent to the extra-fine fiber layer; and
    the fiber layered body is obtained by making a part of the fibers constituting the extra-fine fiber layer to enter into the non-extra-fine fiber layer at the time of bonding the extra-fine fiber layer with the non-extra-fine fiber layer, and by making the fibers constituting the extra-fine fiber layer to be substantially melted in contact to the interface with the non-extra-fine fiber layer, and having:

        a peeling strength as measured as set out in the description between the extra-fine fiber layer and the non-extra-fine fiber layer of 0.40 N/5cm or higher, and
        a surface unevenness as measured as set out in the description relative to a thickness of the fiber layered body of 40% or less.

2. The fiber layered body according to claim 1, wherein the thermoplastic elastomeric fibers are polyurethane-based elastomeric fibers or polystyrene-based elastomeric fibers.

3. The fiber layered body according to claim 1 or 2, wherein the extra-fine fiber layer has a basis weight of 50 g/m$^2$ or less.

4. The fiber layered body according to any one of claims 1 to 3, wherein the non-extra-fine fiber layer contains hydrophilic fibers and hydrophobic fibers.

5. The fiber layered body according to any one of claims 1 to 4, wherein the extra-fine fiber layer is a meltblown nonwoven fabric, and the non-extra-fine fiber layer is a spunlaced nonwoven fabric.

6. The fiber layered body according to any one of claims 1 to 5, which has a water retention ratio as measured as set out in the description of 700 to 1500 mass%.

7. The fiber layered body according to any one of claims 1 to 6, which has a recovery ratio as measured as set out in the description after 25% elongation of 60% or higher when the fiber layered body under wet is elongated in at least one direction.

8. The fiber layered body according to any one of claims 1 to 7, which has a wet friction force of 0.5 to 3 N wherein the wet friction force is measured in accordance with ASTM-D1894 in which the fiber layered body infused with a liquid component at a proportion of 700 mass% based on a mass of the fiber layered body is placed on an acrylic plate in contact to the extra-fine fiber layer.

9. The fiber layered body according to any one of claims 1 to 8, wherein the non-extra-fine fiber layer is in the form of a woven fabric, a knitted fabric, a dry-laid nonwoven fabric, a direct-spun nonwoven fabric, or a web.

10. The fiber layered body according to any one of claims 1 to 9, infused with a liquid component.

11. A face mask comprising the fiber layered body recited in claim 10.

**12.** A liquid-infused sheet comprising the fiber layered body recited in claim 10.

**13.** A method for producing the fiber layered body recited in any one of claims 1 to 9, the method comprising: blowing thermoplastic elastomeric fibers in a molten state onto the non-extra-fine fiber layer to form the extra-fine fiber layer.

**Patentansprüche**

**1.** Faserschichtkörper, umfassend:

eine extrafeine Faser-Schicht, welche thermoplastische elastomere Fasern umfasst und einen durchschnittlichen Faserdurchmesser von weniger als 10 μm aufweist; und
eine nicht-extrafeine Faser-Schicht, welche Fasern mit einem durchschnittlichen Faserdurchmesser von 10 bis 30 μm umfasst und an die extrafeine Faser-Schicht angrenzt; und
wobei der Faserschichtkörper erhalten wird, indem ein Teil der Fasern, welche die extrafeine Faser-Schicht bilden, zu dem Zeitpunkt des Verbindens der extrafeine Faser-Schicht mit der nicht-extrafeine Faser-Schicht in die nicht-extrafeine Faser-Schicht eindringen gelassen werden, und indem die Fasern, die die extrafeine Faser-Schicht bilden, in Kontakt mit der Grenzfläche mit der nicht-extrafeine Faser-Schicht im Wesentlichen schmelzen gelassen werden, und aufweisend:

eine Schälfestigkeit, gemessen wie in der Beschreibung angegeben, zwischen der extrafeine Faser-Schicht und der nicht-extrafeine Faser-Schicht von 0,40 N/5cm oder mehr, und
eine Oberflächenunebenheit, gemessen wie in der Beschreibung angegeben, relativ zu einer Dicke des Faserschichtkörpers von 40% oder weniger.

**2.** Faserschichtkörper nach Anspruch 1, wobei die thermoplastischen elastomeren Fasern elastomere Fasern auf Polyurethanbasis oder elastomere Fasern auf Polystyrolbasis sind.

**3.** Faserschichtkörper nach Anspruch 1 oder 2, wobei die extrafeine Faser-Schicht ein Flächengewicht von 50 g/m$^2$ oder weniger aufweist.

**4.** Faserschichtkörper nach einem der Ansprüche 1 bis 3, wobei die nicht-extrafeine Faser-Schicht hydrophile Fasern und hydrophobe Fasern enthält.

**5.** Faserschichtkörper nach einem der Ansprüche 1 bis 4, wobei die extrafeine Faser-Schicht ein schmelzgeblasener Vliesstoff ist und die nicht-extrafeine Faser-Schicht ein gesponnener Vliesstoff ist.

**6.** Faserschichtkörper nach einem der Ansprüche 1 bis 5, welcher ein Wasserrückhalteverhältnis, gemessen wie in der Beschreibung angegeben, von 700 bis 1500 Massen-% aufweist.

**7.** Faserschichtkörper nach einem der Ansprüche 1 bis 6, welcher ein Erholungsverhältnis, gemessen wie in der Beschreibung angegeben, nach 25 % Dehnung von 60 % oder mehr aufweist, wenn der Faserschichtkörper im nassen Zustand in mindestens eine Richtung gedehnt wird.

**8.** Faserschichtkörper nach einem der Ansprüche 1 bis 7, welcher eine Nassreibungskraft von 0,5 bis 3 N aufweist, wobei die Nassreibungskraft gemäß ASTM-D1894 gemessen wird, wonach der Faserschichtkörper, der mit einer flüssigen Komponente in einem Anteil von 700 Massen-%, bezogen auf eine Masse des Faserschichtkörpers, durchtränkt ist, auf einer Acrylplatte in Kontakt mit der extrafeine Faser-Schicht angeordnet ist.

**9.** Faserschichtkörper nach einem der Ansprüche 1 bis 8, wobei die nicht-extrafeine Faser-Schicht in der Form eines Gewebes, eines Gestricks, eines trockengelegten Vliesstoffes, eines direkt gesponnenen Vliesstoffes oder eines Netzes vorliegt.

**10.** Faserschichtkörper nach einem der Ansprüche 1 bis 9, der mit einer flüssigen Komponente durchtränkt ist.

**11.** Gesichtsmaske, umfassend den Faserschichtkörper nach Anspruch 10.

**12.** Mit Flüssigkeit durchtränktes Blatt, umfassend den Faserschichtkörper nach Anspruch 10.

13. Verfahren zur Herstellung des Faserschichtkörpers nach einem der Ansprüche 1 bis 9, wobei das Verfahren das Blasen thermoplastischer elastomerer Fasern in einem geschmolzenen Zustand auf die nicht-extrafeine Faser-Schicht umfasst, um die extrafeine Faser-Schicht zu bilden.

**Revendications**

1. Corps stratifié de fibres comprenant :

   une couche de fibres extrafine qui comprend des fibres élastomères thermoplastiques et présente un diamètre de fibre moyen de moins de 10 μm ; et
   une couche de fibres non extrafine qui comprend des fibres ayant un diamètre moyen de fibre allant de 10 à 30 μm et est contiguë à la couche de fibre extrafine; et
   le corps stratifié de fibres est obtenu en faisant qu'une partie des fibres constituant la couche de fibres extrafine pénètre dans la couche de fibres non extrafine au moment du collage de la couche de fibres extrafine avec la couche de fibres non extrafine, et en faisant que les fibres constituant la couche de fibres extrafine soient substantiellement en fusion au contact de l'interface avec la couche de fibres non extrafine et ayant :

      une résistance au pelage telle que mesurée comme spécifié dans la description entre la couche de fibres extrafine et la couche de fibres non extrafine de 0,40 N/5 cm ou plus, et
      une irrégularité de surface tel que mesuré comme spécifié dans la description relative à une épaisseur du corps stratifié de fibres de 40% ou moins.

2. Corps stratifié de fibres selon la revendication 1, dans lequel les fibres élastomères thermoplastiques sont des fibres élastomères à base de polyuréthanne ou des fibres élastomères à base de polystyrène.

3. Corps stratifié de fibres selon la revendication 1 ou 2, dans lequel la couche de fibres extrafine a un poids de base de 50 g/m$^2$ ou moins.

4. Corps stratifié de fibres selon l'une quelconque des revendications 1 à 3, dans lequel la couche de fibres non extrafine contient des fibres hydrophiles et des fibres hydrophobes.

5. Corps stratifié de fibres selon l'une quelconque des revendications 1 à 4, dans lequel la couche de fibres extrafine est un tissu non tissé soufflé-fondu et la couche de fibres non extrafine est un tissu non tissé lacé par filage.

6. Corps stratifié de fibres selon l'une quelconque des revendications 1 à 5, lequel présente un rapport de rétention d'eau tel que mesuré comme spécifié dans la description allant de 700 à 1500% en masse.

7. Corps stratifié de fibres selon l'une quelconque des revendications 1 à 6, lequel présente un rapport de récupération tel que mesuré comme spécifié dans la description après un allongement de 25%, de 60% ou plus quand le corps stratifié de fibres sous humidité s'allonge dans au moins une direction.

8. Corps stratifié de fibres selon l'une quelconque des revendications 1 à 7, lequel présente une force de frottement au mouillé allant de 0,5 à 3 N dans lequel la force de frottement au mouillé est mesurée selon la norme ASTM-D1894 dans lequel le corps stratifié de fibres infusé avec un composant liquide à une proportion de 700% en masse, sur base de la masse du corps stratifié de fibres, est placé sur une plaque acrylique en contact avec la couche de fibres extrafine.

9. Corps stratifié de fibres selon l'une quelconque des revendications 1 à 8, dans lequel la couche de fibres non extrafine est sous forme d'un tissu tissé, d'un tissu tricoté, d'un tissu non tissé par voie sèche, d'un tissu non tissé de filature directe ou un voile.

10. Corps stratifié de fibres selon l'une quelconque des revendications 1 à 9, infusé avec un composant liquide.

11. Masque facial comprenant le corps stratifié de fibres décrit à la revendication 10.

12. Feuille infusée de liquide comprenant le corps stratifié de fibres décrit à la revendication 10.

**13.** Procédé de production du corps stratifié de fibres décrit dans l'une quelconque des revendications 1 à 9, le procédé comprenant : le soufflage des fibres élastomères thermoplastiques à l'état fondu sur la couche de fibres non extrafine pour former la couche de fibres extrafine.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2483078 A1
- WO 2011004834 A
- JP 2010155454 A
- JP 2009256856 A